# EUROPEAN PATENT SPECIFICATION

(11) **EP 1 638 518 B1**
(45) Date of publication and mention of the grant of the patent: **10.10.2012**
(21) Application number: 04730426.6
(22) Date of filing: 30.04.2004
(51) Int. Cl.: A61Q 11/00, A61K 8/73

(54) **ORAL CEREAL BETA GLUCAN COMPOSITIONS**
ORALE ZUSAMMENSETZUNGEN AUF DER BASIS VON BETA GLUKAN AUS GETREIDEN
COMPOSITIONS DE BETA-GLUCANE DE CEREALE DESTINEES A ETRE ADMINISTREES PAR VOIE ORALE

(30) Priority: 10.06.2003 US 477048 P
(43) Date of publication of application: 29.03.2006
(73) Proprietor: Ceapro Inc., Edmonton, Alberta T6J 2E1 (CA)
(72) Inventor: REDMOND, Mark, J., Edmonton, Alberta T6C 1B3 (CA); FIELDER, David, A., Edmonton, Alberta T6A 2S6 (CA)
(74) Representative: Harding, Charles Thomas
(86) International application number: PCT/CA2004/000661
(87) International publication number: WO 2004/108103

(56) References cited:
- EP-A- 1 066 823
- WO-A-00/54739
- WO-A-02/02645
- WO-A-02/43719
- WO-A-02/092028
- WO-A-03/054077
- US-A- 5 104 644
- US-A- 6 159 459
- US-A1- 2002 054 917
- US-A1- 2003 091 516

## Description

The present invention relates to tooth paste, nouthrince and tooth whitening compositions comprising β-glucan and to methods of using these compositions, and to methods for importing fresh brecah to a subject ares a prolonged period of time.

### BACKGROUND OF THE INVENTION

Gums are either hydrophobic or hydrophilic substances of molecular weight ranging from 10,000 to 50,000,000 Daltons, which in an appropriate solvent produce gels or highly viscous suspensions or solutions at low dry substance content. Gums commonly used in food, medicine, and industrial products include starches, cellulose derivatives, pullulan, agar, aloe, gellan, guar gum, locust bean gum, pectin, algin, carrageenan, xanthan, β-glucan, and gum arabic (see Whistler, R.L. (1993) Industrial Gums: Polysacchartdes and their derivatives Eds. Whistler R.L. and BeMiller J.N. (Academic Press) p2).

Glucans are homopolysaccharides consisting only of glucose. However, since it is possible to link the glucose molecules in different stereochemical configurations, glucans are a diverse group of compounds with differing chemical, physical, and functional properties.

Chemical structures of polysaccharides are of prime importance in determining their properties. This can be appreciated by comparing the properties of some common homoglucans. For example, cellulose, a P (1-4)-D-glucan, is water insoluble and highly crystalline compared to other polysaccharides. Amylose, an α (1-4)-D-glucan, is sparingly soluble in water, crystallizes less well than cellulose, and can form rigid thermo-reversible gels. Dextran, a (1-6)-α-glucan, with a small degree of branching, is extremely water soluble and non-gel forming. (See Dea, I.C.M. in (1993) Industrial Gums: Polysaccharides and their derivatives Eds. Whistler R.L. and BeMiller J.N. (Academic Press) p21).

O at β (1-3) β (1-4) glucan is classified as a viscous gum, (see Wood, P.J. (1993) Oat Bran Ed P.J. Wood (American Association of Cereal Chemists, Inc. St. Paul, MN)). Cereal β (1-3) β (1-4)-glucans are structural polysaccharides present in the cell walls of cereals, such as barley and oat, among others.

Oat β (1-3) β (1-4) glucan is recognised by the U.S. FDA as an agent that may aid in the prevention of heart disease. In 1997, the FDA allowed oat products to make a health claim. It is important to note that no other β-glucan source, yeast, fungal, bacterial, or cereal is recognised as having these effects. Oat β (1-3) β (1-4)-glucans are therefore distinct.

Unmodified oat β (1-3) β (1-4) glucan forms highly viscous solutions in water at concentrations >0.75%. At concentrations >1.2% the solutions have the consistency of a thick hydrogel.

Glucans of significantly different molecular structure and with different physical and chemical properties compared to oat are found in yeast, fungi, and certain bacteria and genetically engineered bacteria. For example, gellan, polymeric (1-3) β-D-glucopyranosyl [β (1-3)-glucan] produced in *Alcaligenes faecalis* is found in Curdlan (Takeda Chemical Ind. Ltd.), β (1-3) α (1-6) glucopyranoside produced in *Aureobasidium pullulans* is found in pullulan, and β (1-3) β (1-6) glucopyranoside is found in yeast.

The molecular weight of the glucans varies with source. Table 1 shows the average molecular weight of typical gums.

**Table 1: Typical Molecular Weight Range of Common Gums**

| **GUM** | **AVERAGE MOLECULAR WEIGHT** |
|---|---|
| O at β (1-3) β (1-4) glucan | 500,000 - 1,000,000 |
| Pullulan | 50,000 - 100,000 |
| Curdlan | ~500,000 |
| Methyl cellulose | 10,000 - 200,000 |
| Carrageenan | 4,500,000 |
| Xanthan | 15,000,000 - 50,000,000 |
| Sodium alginate | 10,000 - 18,000,000 |

The viscosity of a 1% solution of different polysaccharide gum solutions varies with origin and chemical nature. Table 2 shows the viscosity of 1% solutions of typical gums.

**TABLE 2: Typical Viscosity Ranges of 1% Solutions of Common Gums, Measured at 25°C**

| **GUMS** | **1% SOLUTION VISCOSITY, cP** |
|---|---|
| Oat β(1-3) (1-4) glucan | 500-1500 |
| Pullulan | 2 |
| Gum Arabic | 1-5 |
| Methyl cellulose | 200 |
| Tamarind gum | 100-200 |
| Guar gum | 2,000-3,000 |
| Locust bean gum | 2,000-3,000 |
| Xanthan | 2,000-3,000 |
| Sodium alginate | 200-700 |

The solubility properties of glucans differ according to their source. For example, cereal β (1-3) β (1-4) glucans are normally soluble in aqueous solvents, whereas yeast *(Saccharomyces cerevisiae) β* (1-3) β (1-6)-*glucans* are insoluble in aqueous solvents. Soluble-glucans are desirable. Yeast β-glucan has been solubilized by the addition of phosphate groups (see Williams et al. Immunopharmacol. 22: 139-156 (1991). Jamas et al. (U.S. Patent No. 5,622,939) describes methods to extract soluble β (1-3) β (1-6) glucan from *Saccharomyces cerevisiae.* The method described is complex involving acid hydrolysis, base hydrolysis and the extensive use of centrifugation and ultrafiltration. No details are provided as to the stability of the solubilized yeast β (1-3) β (1-6) glucan.

A number of prior art references disclose methods of preparing-glucans and liquid-glucan compositions from cereals. Among these prior art references are the following:
Beer, et al., Extraction of Oat Gum from Oat Bran: Effects of Process on Yield, Molecular Weight Distribution, Viscosity and (1-3) (1-4) beta-D-Glucan Content of the Gum, Cereal Chemistry 73(1): 58-62 (1996). This reference describes the use of alcohols in an amount equal to or greater than 50% (v/v) to achieve precipitation. The purity of the recovered glucans was reported to be between 22 and 63 %.

Wood et al., Large Scale Preparation and Properties of Oat Fractions Enriched in (1-3)(1-4) beta-D-Glucan, Cereal Chemistry 66(2): 97-103 (1989). This reference describes the use of alcohols in an amount equal to or greater than 50% (v/v) to achieve precipitation of glucans.

U.S. Patent No. 6,323,338 discloses a method of isolating oat β-glucan as an enriched skin from an extract of oat bran. The disclosed method does not utilize low concentrations of short-chain alcohols for the precipitation of the glucan.

Redmond (U.S. Patent No. 6,284,886) discloses compositions of cereal β (1-3) β (1-4) glucans, and methods of producing these compositions. The disclosed compositions meet the strict requirements of the cosmetics industry, in terms of their viscosity, shear strength, and moisture-enhancing properties. No method for the extraction or purification of β (1-3) β (1-4) glucan is described.

U.S. Patent No. 6,159,459 discloses toothpaste and mouthwash compositions comprising Scleroghican, a polymer having a triple helical β (1-3) linked glucopyranose backbone with a single β (1-6) linked glucopyranosyl branch every third subunit. The Soleroglucan composition is disclosed as being useful in the treatment of xerostamia.

U.S. Patent No. 5,980,918 discloses topical compositions comprising a cereal beta glucan and an antimicrobial agent selected from the group consisting of methyl paraben, ethyl paraben, butyl paraben and propyl paraben.

U.S. Patent Application No. 09/973,185, published as U.S. Publication No. 20020102316, discloses an anti-snoring composition comprising oat beta glucan, glycerin as a humectant, extract of Prickly Ash antibacterial agent, Polysorbate 80 and Polysorbate 20 as surfactants, and flavouring agents. '

U.S. Patient Application No. 10/021970, published as U.S. Publication No. 20020054917, discloses a dietary supplement composition comprising oat beta glucan, lactoferrin as an antimicrobial agent, a lemon flavouring, mannitol, and sorbitol.

### SUMMARY OF THE INVENTION

The present invention is summarised in the numbered paragraphs below.
1. A toothpaste for imparting fresh breath to a subject over a prolonged period of time, comprising:
   an effective amount of a β (1-3) β (1-4) glucan;
   an effective amount of an antibacterial agent;
   an effective amount of a flavouring agent;
   an effective amount of a surfactant, and
   an effective amount of a polishing agent.
2. The toothpaste according to paragraph 1, wherein the toothpaste comprises:
   an effective amount of a β (1-3) β (1-4) glucan;
   an effective amount of an antibacterial agent;
   an effective amount, of a flavouring agent;
   an effective amount of a surfactant;
   an effective amount of a polishing agent, and
   an effective amount of a fluoride salt.
3. The toothpaste according to paragraph 1, wherein the toothpaste comprises:
   an effective amount of a β (1-3) β (1-4) glucan;
   an effective amount of an antibacterial agent;
   an effective amount of a flavouring agent;
   an effective amount of a surfactant;
   an effective amount of a sweetener;
   an effective amount of a polishing agent, and
   an effective amount of a fluoride salt.
4. The toothpaste according to paragraphs 1, 2 or 3, wherein the antibacterial agent is selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.
5. A mouthrinse for imparting fresh breath to a subject over a prolonged period of time, comprising:
   an effective amount of a β (1-3) β (1-4) glucan;
   an effective amount of an antibacterial agent;
   an effective amount of a flavouring agent;
   an effective amount of a surfactant, and
   an effective amount of a sweetener.
6. The mouthrinse according to composition of paragraph 5, wherein the antibacterial agent is selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.
7. A tooth-whitening composition comprising:
   an effective amount of a β (1-3) β (1-4) glucan, and
   an effective amount of a bleaching agent.
8. The tooth-whitening composition according to paragraph 7, further comprising an effective amount of one, or more than one compound selected from the group consisting of a flavouring agent, an antibacterial agent, a botanical extract, a surfactant, a humectant, a thickener, a fluoride salt, an odour neutralizing agent, an antioxidant, and a polishing agent.
9. The tooth-whitening composition according to paragraph 7, comprising:
   an effective amount of a β (1-3) β (1-4) glucan;
   an effective amount of a flavouring agent, and
   an effective amount of a bleaching agent.
10. The tooth-whitening composition according to paragraph 7, comprising:
   an effective amount of a β (1-3) β (1-4) glucan;
   an effective amount of a flavouring agent;
   an effective amount of an antibacterial agent, and
   an effective amount of a bleaching agent.
11. A mouthrinse for imparting fresh breath to a subject over a prolonged period of time, comprising:
   an effective amount of a β (1-3) β (1-4) glucan, and
   an effective amount of an odour neutralizing agent.
12. The mouthrinse according to paragraph 11, further comprising an effective amount of one, or more than one compound selected from the group consisting of a flavouring agent, an antibacterial agent, a botanical extract, a surfactant, a humectant, a thickener, a fluoride salt, a bleaching agent, an antioxidant, and a polishing agent.
13. The mouthrinse according to paragraph 11, wherein said odour neutralizing agent is selected from the group consisting of zinc gluconate, zinc citrate, alpha ionone, and a mixture thereof.
14. A mouthrinse for imparting fresh breath to a subject over a prolonged period of time, comprising:
   an effective amount of a β (1-3) β (1-4) glucan;
   an effective amount of an antibacterial agent selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.
15. The mouthrinse according to paragraph 14, further comprising an effective amount of one, or more than one compound selected from the group consisting of a flavouring agent, a polishing agent, a surfactant, a botanical extract, a humectant, a thickener, a fluoride salt, a bleaching agent, a gum base, an antioxidant, and an emulsifier.
16. A method for imparting fresh breath to a subject over a prolonged period of time, comprising applying to the teeth, the oral cavity, or both of a subject, a composition comprising:
   an effective amount of a β (1-3) β (1-4) glucan, and
   an effective amount of one, or more than one of an antibacterial agent, a botanical extract, and a flavouring agent.
17. The method according to paragraph 16, wherein the composition further comprises an effective amount of one, or more than one compound selected from the group consisting of a surfactant, a sweetener, a polishing agent, a thickener, a fluoride salt, a bleaching agent, a humectant, an odour neutralizing agent, an antioxidant, and a gum base.
18. A method of whitening the teeth of a subject, comprising applying to the teeth of the subject an oral composition comprising:
   an effective amount of a β (1-3) β (1-4) glucan, and
   an effective amount of a bleaching agent.
19. The method according to paragraph 18, wherein the oral composition further comprises an effective amount of one, or more than one compound selected from the group consisting of a flavouring agent, an antibacterial agent, a botanical extract, a surfactant, a humectant, a thickener, a fluoride salt, an antioxidant, and a polishing agent.

The present description relates generally to cereal β-glucan compositions. More particularly, the present description relates to oral cereal β-glucan compositions for freshening the breath of a subject, and to methods of using these compositions.

In one aspect, the description provides an oral composition comprising:
an effective amount of a β (1-3) β (1-4) glucan;
an effective amount of an antibacterial agent, or a botanical extract, or both;
an effective amount of a flavoring agent,
an effective amount of a surfactant, and
an effective amount of one, or more than one compound selected from the group consisting of a sweetener, a polishing agent, a thickener, a fluoride salt, a bleaching agent, an odour neutralizing agent, an antioxidant, and a gum base.

The above-defined oral composition may further comprise a humectant.

In one embodiment of the description, the above-defined oral composition comprises:
an effective amount of a β (1-3) β (1-4) glucan;
an effective amount of an antibacterial agent;
an effective amount of a flavoring agent;
an effective amount of a surfactant, and
an effective amount of a sweetener.

In another embodiment of the description, the above-defined oral composition comprises:
an effective amount of a β (1-3) β (1-4) glucan;
an effective amount of an antibacterial agent;
an effective amount of a flavoring agent,
an effective amount of a surfactant, and
an effective amount of a polisbing agent.

In a further embodiment of the description, the above-defined oral composition comprises:
an effective amount of a β (1-3) β (1-4) glucan;
an effective amount of an antibacterial agent;
an effective amount of a flavoring agent;
an effective amount of a surfactant;
an effective amount of a polishing agent, and
am effective amount of a fluoride salt.

In an even further embodiment of the description, the above-defined oral composition comprises:
an effective amount of a β (1-3) β (1-4) glucan;
an effective amount of an antibacterial agent;
an effective amount of a flavoring agent;
an effective amount of a surfactant;
an effective amount of a sweetener;
an effective amount of a polishing agent, and
an effective amount of a fluoride salt.

In a second aspect, the present description provides an oral composition comprising:
an effective amount of a β (1-3) β (1-4) glucan;
an effective amount of an antibacterial agent, or a botanical extract, or both;
an effective amount of a flavoring agent;
an effective amount of one; or more than one compound selected from the group consisting of a polishing agent, a thickener, a fluoride salt, a bleaching agent, an odour neutralizing agent, an antioxidant, and a gum base.

The oral composition of the second aspect of the description may further comprise one, or more than one of a humectant, a surfactant, and a sweetener.

in a third aspect, the present description provides an oral composition comprising:
an effective amount of a β (1-3) β (1-4) glucan, and
an effective amount of a polishing agent.

The oral composition of the third aspect of the present description may further comprise an effective amount of one, or more than one compound selected from the group consisting of a sweetener, a flavoring agent, an antibacterial agent, a botanical extract, a surfactant, a humectant, a thickener, a fluoride salt, an odour neutralizing agent, an antioxidant, and a bleaching agent.

In a particular example, the oral composition of the third aspect of the present description composes: comprises:
an effective amount of a β (1-3) β (1-4) glucan;
an effective amount of a flavoring agent;
an effective amount of an antibacterial agent, and
an effective amount of a polishing agent.

In a fourth aspect, the present description provides an oral composition comprising:
an effective amount of a β (1-3) β (1-4) glucans, and
an effective amount of a bleaching agent.

The oral composition of the fourth aspect of the present description may further comprise an effective amount of one, or more than one compound selected from the group consisting of a flavoring agent, an antibacterial agent, a botanical extract, a surfactan, a humectant, a thickener, a fluoride salt, an odour neutralizing agent, an antioxidant, and a polishing agent

In one example, the oral composition of the fourth aspect of the present description comprises:
an effective amount of a β (1-3) β (1-4) glucan;
an effective amount of a flavouring agent, and
an effective amount of a bleaching agent.

In another example, the oral composition of the fourth aspect of the present description comprises:
an effective amount of a β (1-3) β (1-4) glucan;
an effective amount of a flavouring agent;
an effective amount of an antibacterial agent, and
an effective amount of a bleaching agent.

In a fifth aspect, the present description provides an oral composition comprising:
an effective amount of a β (1-3) β (1-4) glucan, and
an effective amount of an odour neutralizing agent.

The oral composition of the fifth aspect of the present description may further comprise an effective amount of one, or more than one compound selected from the group consisting of a flavoring agent, an antibacterial agent, a botanical extract, a surfactant, a humectant, a thickener, a fluoride salt, a bleaching agent, an antioxidant, and a polishing agent.

Preferably, the odour neutralizing agent is selected from the group consisting of zinc gluconate, zinc citrate, alpha ionone, and a mixture thereof.

In an embodiment of the above-defined oral compositions, the antibacterial agent is selected from the group consisting of triclosan, cetyl Pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, merithol, and a mixture thereof

In a sixth aspect, the present description provides an oral composition comprising:
an effective amount of a β (1-3) β (1-4) glucan;
an effective amount of an antibacterial agent selected from the group consisting of triclosan, cetyl pyndinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.

The oral composition of the sixth aspect of the present description may further comprise an effective amount of one, or more than one compound selected from the group consisting of a flavouring agent, a polishing agent, a surfactant, a humectant, a thickener, a fluoride salt, a bleaching agent, an antioxidant, a botanical extract, and a gum base.

In another embodiment of the above-defined oral compositions, the flavoring agent is selected from the group consisting of a synthetic flavour, an aromatic oil, an oleo resin, a plant-derived extract, a flower-derived extract, a fruit-derived extract, and a mixture thereol.

In a seventh aspect, the present description provides a method of producing fresh breath in a subject, comprising applying to the teeth, the oral cavity, or both of the subject, a composition comprising:
an effective amount of a β (1-3) β (1-4) glucan, and
an effective amount of one, or more than one of an antibacterial agent,
a botanical extract, and a flavouring agent.

In an embodiment of the method of the seventh aspect of the present description the composition further comprises an effective amount of one, or more than one compound selected from the group consisting of a surfactant, a sweetener, a polishing agent, a thickener, a fluoride salt, a bleaching agent, a humectant, an odour neutralizing agent, an antioxidant, and a glum base.

In one example, the composition of the method of the seventh aspect of the present description comprises:
an effective amount of a β (1-3) (1-4) glucan, and
an effective amount of a flavouring agent.

In another example, the composition of the method of the seventh aspect of the present description comprises:
an effective amount of a β (1-3) β (1-4) glucan, and
an effective amount of a flavouring agent, and
an effective amount of an antibacterial agent.

In an eighth aspect, the present description provides a method of whitening the teeth of a subject, comprising applying to the teeth of the subject, an oral composition comprising:
an effective amount of a β (1-3) β (1-4) glucan, and
an effective amount of a bleaching agent.

In an embodiment of the method of the eighth aspect of the present description, the oral composition further comprises an effective amount of one, or more than one compound selected from the group consisting of a flavoring agent, an antibacterial agent, a botanical extract, a surfactant, a humectant, a thickener, a fluoride salt, an antioxidant, and a polishing agent.

In another embodiment of the above-defined compositions of the present description the botanical extract is an extract of Guarana, *Ginkgo biloba*, Kola nut, Goldenseal, Golo Kola, *Schizandra*, Elderberry, St. John's Wort, Valerian and *Ephedra,* black tea, white tea, java tea, garlic oil, fiber, green tea, lemon oil, mace, licorice, onion oil, orange oil, rosemary, milk thistle, *Echinacea*, Siberian ginseng or *Panax ginseng*, *lemon balm*, *Kava kava*, matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, or passion flower..

The toothpaste, mouth risse and tooth writereng compositions of the present invention are advantageous in that they are retained on the teeth, or in the oral cavity of a subject, or both, over a prolonged period of time, and, can, therefore, impart long-lasting fresh breath to the subject by continuously providing an antibacterial agent for killing odour-causing bacteria, or a flavouring agent for providing a favourable taste sensation to the subject, or both. The may also include one, or more than one odour-neutralizing agent for neutralizing sulfur-products produced by the odour-causing bacteria When applied to the teeth of a subject, the compositions of the present invention also produce a pleasing smooth, clean feel.

The tooth whitering compositions of the present invention, which contain a bleaching agent are advantageous over prior art bleaching compositions, in that they can be retained on the surface of the teeth of a subject over a relatively longer period of time, and, as a result, can be more clinically effective in whitening the teeth of the subject

The tooth paste, nocthrinse and both whitening compositions of the present invention, which comprise nutraceuticals (for example, botanical extracts), or medically active compounds, do not require alcohol for solubilization of the active components, and are, therefore, suitable for use by individuals who can not consume alcohol for health or religious reasons.

### DESCRIPTION OF PREFERRED EMSODIMENT

The present invention relates to toothpaste, noathrinse and tooth whiptering compositions comprising β-glucan and to methods of using these compositions. The present invention also particularly relates to tooth paste, north rinse and tooth whitering compositions comprising a β (1-3) β (1-4) glucan and a botanical extract

The practice of the present invention will employ, unless otherwise indicated, conventional methods of chemistry, cereal chemistry, and biochemistry, within the skill of the art. Such techniques are explained fully in the literature. See for example, industrial Gums: Polysaccharides and their derivatives Eds. Whistler RL and BaMiller J.N. (Academic Press), Oats: Chemistry and Technology ed. Webster FH (American Association of Cereal Chemists, St. Paul, MN).

As used in this specification and the appended claims, the singular forms "a", "an" and "the" include plural references unless the content clearly indicates otherwise.

### Definitions

In describing the present invention, the following terms will be employed, and are intended to be defined as indicated below.

By "cereal" is meant any of several grains such as, but not limited to, cultivars of barley, oat, wheat, rye, sorghum, millet, and corn.

By "glycin" is meant a polymer of monosaccharides linked together by glycosidic bonds.

By "glucan" is meant a homopolysaccharide consisting only of glucose.

By "cereal beta glucan" is meant a glucan with a β (1-3)-linked glucopyranosyl backbone, or a β (1~4)-linked glucopyranosyl backbone, or a mixed β (1-3) β (1-4)-linked glucopyranosyl backbone, which is derived from a cereal source.

By "β (1-3) β (1-4) glucan" is meant a cereal β glucan.

By "gum" is meant a plant or microbial polysaccharide or their derivatives that are dispersible in either cold or hot water to produce viscous mixtures or solutions. Gums may be classified by origin: exudate gums, seaweed gums, seed gums, starch and cellulose derivatives, and microbial gums.

By "compound of interest" is meant any pharmaceutical, medicinal, therapeutic, flavouring, sweetener or other functional materials mixed with the β (1-3) β (1-4) glucan to produce a coatable composition.

By "effective amount" is meant the amount of the one, or more than one compound of interest necessary to achieve a desired effect, such as a physiological effect, or a stimulatory effect.

By "sequester" is meant the incorporation, entrapment, or solubilization of hydrophilic compounds, or hydrophobic compounds, for example, small molecular weight hydrophobic compounds, such as essential oils, pharmaceutical, medicinal, and therapeutic agents.

By "surfactant" is meant a surface-active agent, which stabilizes a mixture of immiscible or insoluble materials by reducing interfacial tension at the boundaries between the two materials. The surfactant surrounds and separates the dispersed medium, and prevents it from forming large droplets or particles, which can separate into a second phase.

By "odour neutralizing agent" is meant a compound that can neutralize the odour of sulphur compounds, or other odiferous volatile products within the mouth of a subject.

By "humectant" is meant a compound for controlling the moisture level in the compositions of the present invention.

By "bleaching agent" is meant a compound effective in whitening the surface of teeth.

By "antioxidant" is meant a compound that can counteract the damaging effects of oxygen, and, more particularly, free-radical molecules, in tissues. Non-limiting examples include beta-carotene, lycopene, and vitamin E.

The compositions of the present invention can be formed by mixing an aqueous solution comprising about 0.01 wt. % to about 20 wt. % of the β (1-3) β (1-4) glucan with one, or more than one compound of interest, such as an antibacterial agent, or a flavouring agent. It is preferred that the resulting compositions be left undisturbed after being mixed for a period of time sufficient to allow the formation of a homogeneous composition in the form of a suspension, emulsion or gel. In many cases, the amount of time required to obtain a homogeneous composition is from about eight to about 16 hours. It is to be appreciated, however, that shorter or longer periods of time may be required, depending on the quantity and purity of the β (1-3) β (1-4) glucan used, as well as on the quantity and nature of each of the one, or more than one compound of interest. Compositions of the present invention, which are in the form of a gel may be converted into a more fluid state by gentle agitation.

Without wishing to be bound by theory, the formation of the homogeneous suspension, emulsion or gel is believed to be caused by the one, or more than one compound of interest being sequestered or encapsulated within the β (1-3) β (1-4) glucan, and subsequent formation of hydrogen bonds between molecules of the one, or more than one compound of interest and the β (1-3) β (1-4) glucan. Another possibility is that the β (1-3) β (1-4) glucan acts as a surfactact or emulsifying agent by reducing the interfacial tension at the boundaries between the one, or more than one compound of interest and the aqueous phase within which the β (1-3) β (1-4) glucan is dispersed, and, consequently, effectively solubilizes the one, or more than one compound within the aqueous phase.

The aqueous solution used in the method of making the compositions of the present application preferably contains the beta glucan in an amount of from about 0.01 wt. % to about 1.2% wt. %, more preferably about 0,1 wt. % to about 1.1 wt. %, most preferably about 0.5 wt. % to about 1 wt. %.

The beta glucan solution is preferably mixed with about 0.41 wt. % to about 40 wit. %, preferably about 0.01 wt. % to about 25 wt. %, more preferably about 0.01 wt% to about 4 wt. %, even more preferably about 0.1, wt. % to about 1.4 wt. %, most preferably about 0.5 wt. % to about 1.2 wt. % of the one, or more than one compound of interest.

It is preferred that the beta glucan solutions used in preparing the compositions of the present invention be prepared from a beta glucan having a purity of from about 65% to about 100%, more preferably from about 75% to about 100%, most preferably from about 85% to about 100%. It is particularly preferred that the beta glucan contain less than 20%, more preferably less than 15%, even more preferably less than 10%, most preferably less than 5% of impurities, such as protein, lipid, carbohydrate, and particulate impurities.

Cereal beta glucans suitable for use in the method of making the compositions of the present invention are available in powdered form from several commercial suppliers, such as Sigma Chemical Co. (St. Louis, MO) and Ceapro Inc. (Edmonton, AB, Canada). Solutions of beta glucan can be prepared in the manner described in U.S. Patent No. 6,284,886.

The oral compositions of the present invention can be in the form of a mouthrinse, a tooth-whitening formulation of a toothpaste

To prepare the mouthrinse, or spray compositions of the present invention, the components of the composition are dissolved in water and/or alcohol. To prepare the toothpaste compositions of the present invention, a first solution is first formed containing all water-soluble components, and, afterwards, the water-insoluble components are added. Alternatively, the water-insoluble components may be premixed together, and added as a second phase to the first phase containing the water soluble components of the composition. The two phases may then be blended together.

An example of a mouthrinse composition according to the invention of the present invention comprises:
an effective amount of a β (1-3) β (1-4) glucan;
an effective amount of an antibacterial agent, or a botanical extract, or both;
an effective amount of a flavoring agent,
an effective amount of a surfactant, and
an effective amount of a sweetener.

Another example of a mouthrinse composition according to the present invention comprises:
an effective amount of a β (1-3) β (1-4) glucan;
an effective amount of an antibacterial agent, or a botanical extract, or both;
an effective amount of a flavoring agent;
an effective amount of an odour neutralizing agent.

A further example of a mouthrinse composition according to the present invention comprises:
an effective amount of a β (1-3) β (1-4) glucan, and
an effective amount of an odour neutralizing agent.

An even further example of a mouthrinse composition according to the present invention comprises:
an effective amount of a β (1-3) β (1-4) glucan;
an effective amount of an antibacterial agent selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.

An example of a toothpaste composition according to the present invention comprises:
an effective amount of a β (1-3) β (1-4) glucan;
an effective amount of an antibacterial agent;
an effective amount of a flavoring agent,
an effective amount of a surfactant, and
an effective amount of a polishing agent.

A further example of the a toothpaste composition according to the present invention comprises:
an effective amount of a β (1-3) β (1-4) glucan;
an effective amount of an antibacterial agent;
an effective amount of a flavoring agent;
an effective amount of a surfactant;
an effective amount of a polishing agent, and
an effective amount of a fluoride salt.

Another example of a toothpaste composition according to the present invention comprises:
an effective amount of a β (1-3) β (1-4) glucan;
an effective amount of an antibacterial agent;
an effective amount of a flavoring agent;
an effective amount of a surfactant;
an effective amount of a sweetener;
an effective amount of a polishing agent, and
an effective amount of a fluoride salt.

A further example of the toothpaste composition of the present invention comprises:
an effective amount of a β (1-3) β (1-4) glucan, and
an effective amount of a polishing agent.

Another example of the toothpaste composition of the present description comprises;
an effective amount of a β (1-3) β (1-4) glucan;
an effective amount of a flavoring agent, and
an effective amount of a polishing agent.

A further example of the toothpaste composition of the present description comprises:
an effective amount of a β (1-3) β (1-4) glucan;
an effective amount of a flavoring agent;
an effective amount of an antibacterial agent, and
an effective amount of a polishing agent.

An example of a tooth-whitening formulation according to the present invention comprises:
an effective amount of a β (1-3) β (1-4) glucan, and
an effective amount of a bleaching agent.

A further example of a tooth-whitening formulation according to the present invention comprises:
an effective amount of a β (1-3) β (1-4) glucan;
an effective amount of a flavouring agent, and
an effective amount of a bleaching agent.

Another example of a tooth-whitening formulation according to the present invention comprises:
an effective amount of a β(1-3) β (1-4) glucan;
an effective amount of a flavouring agent;
an effective amount of an antibacterial agent, and
an effective amount of a bleaching agent.

It is preferred that the tooth-whitening composition of the present invention be formulated as a paste or gel for application directly to the surface of the teeth of a subject. Mouthrinse compositions of the present invention containing a consumable botanical extract, such as an extract of Echinacea, may be expectorated, or swallowed.

Also herein is described pharmaceutical composition comprising:
an effective amount of a β (1-3) β (1-4) glucan, and
an effective amount of a botanical extract, or a pharmaceutically active agent.

Examples of the botanical extract that may be used in the pharmaceutical compositions according to the present description include, without limitation, extracts of Guarana, *Ginkgo biloba,* Kola nut, Goldenseal, Golo Kola, *Schizandra*, Elderberty, St. John's Wort, Valerian and *Ephedra*, black tea, white tea, Java tea, garlic oil, fiber, green tea, lemon oil, mace, licorice, onion oil, orange oil, rosemary, milk thistle, *Echinacea,* Siberian ginseng or *Panax ginseng*, lemon balm, *Kava kava*, matte, bilberry, soy, grapefnuit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, and passion flower.

Preferably, the botanical extract is an extract of an oat grain. More preferably, the botanical extract is an oat grain extract, which contains avenanthramide.

As an example of a pharmaceutically active agent there is mentioned an antihistamine, a decongestant, a corticosteroid, a non-steroidal anti-inflammatory drug, a bronchodilator, a vasodilator, or a local anaesthetic. The pharmaceutical composition of the present description can be used in the form of a spray, a liquid, preferably in the form of drops, or as a gel. In a preferred embodiment, the botanical extract, and the pharmaceutically active agent comprise compounds that are readily absorbed through the mucosa of the oral cavity, the mucosa of the nasal cavity, or through gum tissue.

It is preferred that the pharmaceutical compositions of the present description containing an anesthetic be applied to a specific, localized region of the gums or a surface of the oral cavity of a subject. It is also preferred that the compositions of the present description, which contain a vasodilating agent, such as nitroglycerin, be applied underneath the tongue of a subject. The pharmaceutical compositions of the present description, which comprise an antihistamine, a decongestant, a corticosteroid, or a non-steroidal anti-inflammatory drug are preferably applied to the back of the oral cavity, or to the nasal cavity of a subject to allow medication released from the composition to be inhaled by the subject. Pharmaceutical compositions according to the present description which comprise a consumable botanical extract, may be used as a mouthrinse and expectorated after being used, or, alternatively, may be swallowed,

The tooth paste, nonth rinse and booth whitening compositions of the present invention can be retained on the surface of the oral cavity, the teeth or the gums of a subject due to the inherent stickiness property of the β (1-3) β (1-4) glucan. After being applied, the one, or more than one compound of interest is gradually from the tooth paste, nonth rinse and booth whitening composition. The rate of release of the one, or more than one compound of interest can be reduced by increasing the viscosity of the tooth paste, nonth rinse and booth whitening compositions of the present invention through the use of a thickener.

Non-limiting examples of the bleaching agent that can be used in the tooth-whitening compositions of the present invention include hydrogen peroxide and carbamide peroxide.

As examples of antioxidants there are mentioned Vitamin E, Vitamin C, butylated hydroxyanisole (BHA), butylated hydroxytoluene (BHT), propyl gallate, octyl gallate and deodecyl gallate, and tert-butylhyroquinone (TBHQ).

The flavourings that can be used include those known to the skilled artisan, such as natural and artificial flavours. These flavourings may be chosen from synthetic flavours, oils and flavouring aromatics, and/or oils, oleo resins and extracts derived from plants, leaves, flowers, fruits and so forth, and combinations thereof. Representative flavouring oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavours such as vanilla, chocolate, coffee, cocoa and citrus oil, including lemon, orange, grape. lime and grapefruit and fruit essence including apple, pear, peach, strawberry, raspberry, cherry, plum, pineapple, apricot, and so forth. These flavourings can be used individually or in a mixture. Commonly used flavours include mints, for example peppermint and/or spearmint, artificial vanilla, cinnamon derivatives, and various fruit flavours, whether employed individually or in combination. Flavourings such as aldehydes and esters including cinnamyl acetate, cinnamaldehyde, citral, diethylacetal, dihydrocarvyl acetate, eugenyl formate, p-methylanisole, and so forth may be used. Generally, any flavouring or food additive, such as those described in Chemicals Used in Food Processing, publication 1274 by the National Academy of Sciences, pp 63-258. Further examples of aldehydes include, but are not limited to acetaldehyde (apple); benzaldehyde (cherry, almond); cinnamic aldehydes (Cinnamon); citral i.e. (lemon, lime); neral i.e. beta citral (lemon, lime); decanal (orange, lemon); ethyl vanillin (vanilla, cream); heliotropine, i.e. piperonal (vanilla, cream); vanillin (vanilla, cream); alpha-amyl cinnamaldehyde (spicy fruity flavours); butyraldehyde (butter, cheese); valeraldehyde (butter, cheese); citronellal; decanal (citrus fruits); aldehyde C-8 (citrus fruits); aldehyde C-9 (citrus fruits), aldehyde C-12 (citrus fruits); 2-ethyl butyraldehyde (berries); hexanal i.e. trans-2 (berries); tolyl aldehyde (cherry, almond); veratraldehyde (vanilla); 2,6-dimethyl-5-heptenal, i.e. melonal (melon); 2-6-dimethyloctanal (green fruit); and 2-dodecenal (citrus, mandarin); cherry, grape; and mixtures thereof.

The amount of flavouring used is normally a matter of preference subject to such factors as flavour type, individual flavour, and strength desired. Thus, the amount may be varied in order to obtain the result desired in the final product. Such variations are within the capabilities of those skilled in the art without the need for undue experimentation. In general, amounts of about 0.1 to about 30 wt. % are usable with amounts of about 2 to about 25 wt. % being preferred and amounts from about 8 to about 12 wt. % are more preferred.

In general, an amount of sweetener used will depend on the specific sweetener that is selected. This amount will normally be 0.01 % to about 10 % by weight of the composition when using an easily extractable sweetener. The selection and amount of sweetener used is within the capabilities of those skilled in the art without the need for undue experimentation. Other considerations will include the interaction with flavouring.

Sweeteners will be selected either individually or as combinations of sweeteners selected from the following groups:
1. Water-soluble sweeteners are well known in the art. These include, but are not limited to, monosaccharides, disaccharides, and polysaccharides such as xylose, ribose, glucose, dextrose, mannose, galactose, fructose (levulose), sucrose (table sugar), maltose, invert sugar (glucose and fructose) partially hydrolysed starch, corn syrup solids, dihydrochalcones, monellin, steviosides, and glycyrrhizin.
2. Artificial sweeteners such as soluble saccharin salts, cyclamate salts, the sodium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like.
3. Dipeptide based sweeteners, such as L-aspartic acid derived sweeteners, such as L-aspartyl-L-phenylalanine methyl ester (aspartame) and materials describe in U.S. Patent No. 3,492,131. L-alpha-aspartyl-N-(2,2,4,4-tetramethyl-3-thietanyl) -D-alaninamide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclohexyen)-alanine, and the like.
4. Water soluble sweeteners derived from naturally occurring water-soluble sweeteners, such as a chlorinated derivative of sucrose (Sucralose).
5. Protein based sweeteners such as *Thaumatoccous danielli* (Thaumatin I and II).

Surfactants that may be used in the tooth paste, nonth rinse and booth whitening compositions of the present invention include mono and diglycerides of fatty acids and polyoxyethylene sorbitol esters, for example, Atmos 300 and Polysorbate 80. Other examples include alkali metal alkyl sulfates of 8 to 20 carbon atoms, preferably of 10 to 18 and more preferably of 12 to 16 carbon atoms in the alkyls thereof such as sodium lauryl sulfate, sodium lauryl phosphate, sodium cocomonoglyceride sulfate, sodium linear tridecylbenzene sulfonate, N-lauroyl and N-memyl taurate. The amount of surfactant added is typically in the range of about 0.1 to about 15% by weight and preferably about 0.4 to about 5% by weight of the composition. Other surfactants useful in preparing the toothpaste, nouthring and tooth whintering compositions of the present invention include non-ionic organic surface active polymers such as polyoxyethylene-polyoxypropylene block copolymers such as Pluronic 108 and Pluronic F-127 marketed by BASF. Pluronic 108 has a molecular weight of 3200 and contains 80% of the hydrophilic polyoxyethylene moiety and Pluronic F127 has a molecular weight of 4000 and contains 70% polyoxyethylene. Other surfactants will be known to those skilled in the art and may readily be employed without undue experimentation. The surfactants help to disperse components of the composition of the present ingredients throughout the oral cavity.

The compositions of the present invention can also include thickening agents. If thickening agents are present, they can include silica thickeners, carob bean gum, methylcellulose, carrageenan gum, hydroxymethyl cellulose, hydroxypropyl cellulose, hydroxypropyl carboxy methylcellulose (Methocel), or carboxy methylcellulose (CMC), and the like, are typically used in amounts ranging from about 0 to about 5% by weight, preferably about 0.01 to about 0.7% by weight of the composition.

Saliva stimulating agents can also be added to the oral compositons according to the present description Useful saliva stimulating agents are those disclosed in U.S. Patent No. 4,820,50 . Saliva stimulating agents include food acids such as citric, lactic, malic, succinic, ascorbic, adipic, fumaric, and tartaric acids. Preferred food acids are citric, malic, and ascorbic acids. The amount of saliva stimulating agents in the composition is preferably from about 0.01 to about 12% by weight, preferably about 1 to about 10% by weight, even more preferably about 2.5% by weight, to about 6 by weight.

For the oral compositions of the present description it may be desirable to provide a cooling sensation following application of the composition. Preferred cooling agents include mono-menthyl succinate, in amounts ranging from about 0.001 to about 2.0 wt. %, preferably about 0.2 to about 0.4 wt. %. A mono-menthyl succinate containing cooling agent is available from Mane, Inc. Other suitable cooling agents include WS3, WS23, Ultracool II, and the like.

Examples of polishing agents useful to prepare the toothpaste compositions of the present invention include finely divided silica, dicalcium phosphate, calcium pyrophosphate, sodium bicarbonate, insoluble, sodium metaphosphate and tricalcium phosphate.

Examples of humectants, which can be used in the toothpaste, mouthrinse and tooth whitening compositions of the present invention include glycerol, sorbitol, propylene, glycol, polypropylene glycol and/or mannitol.

The compositions of this invention can contain colouring agents or colourings. The colouring agents are used in amounts effective to produce the desired color. The colouring agents useful in the present invention, include pigments such as titanium dioxide, which may be incorporated in amounts of up to about 5 wt. %, and preferably less than about 1 wt. %. Colourings can also include natural food colours and dyes suitable for food, drug and cosmetic applications. These colorants are known as FD&C dyes and lakes. The materials acceptable for the foregoing spectrum of use are preferably water-soluble, and include FD&C Blue No. 2, which is the disodium salt of 5,5-indigotindisulfonic acid. Similarly, the dye known as Green No. 3 comprises a triphenylmethane dye and is the monosodium salt of 4-[4-N-ethyl-p-sulfobenzylamino) diphenyl-methylene-1-[1-N-ethyl-N-p-sulfonium benzyl)-2,5-cyolo-hexadienimine]. A full recitation of all FD&C and D&C dyes and their corresponding chemical structures may be found in the Kirk-Other Encyclopaedia of Chemical Technology, Volume 5, pp 857-884, which text is accordingly incorporated herein by reference.

Examples of solvents that can be used in the toothpaste, mouthrinse and tooth whitening compositions of the present invention include, without limitation, water, and alcohols, such as ethanol.

It is also possible to include preservatives in the composition of the present invention, such as those approved for human consumption and pharmaceutical use, for example, potassium sorbate, sorbic acid, benzalkonium chloride, and parabens.

The beta glucan content of the compositions of the present application can be determined using a number of methods, known to those skilled in the art. For example, beta glucan content can be assessed colorimetrically and/or by standard analytical techniques such as size exclusion chromatography and HPLC (see Wood et al. Cereal Chem. (1977) 54:524; Wood et al. Cereal Chem. (1991) 68:31-39; and Wood et al. Cereal Chem. (1991) 68:530-536). Beta glucans can also be analyzed enzymatically using commercially available kits, such as Megazyme (Ireland) employing the techniques of McCleary and Glennie-Holmes J. Inst. Brew. (1985) 91:285.

Viscosities can be measured with a rotational, shear-type viscometer such as the Brookfield Syncro-Lectric or the Haake Rotovisco. Methods of using the instrument are known to those skilled in the art. Routinely, measurements are made at four speeds of disc rotation at a constant temperature of 25°C.

The following examples are provided to exemplify the present invention. Variations and alterations will be readily apparent to those skilled in the art.

### Materials

A 1 wt. % aqueous solution of oat beta glucan having a purity of >85% was used in the following examples. This solution was either purchased directly from Ceapro Inc. (Edmonton, AB, Canada), or prepared from an oat beta glucan powder (>85% purity) also obtained from Ceapro Inc. Solutions were prepared from the beta glucan powder in the manner described in U.S. Patent No. 6,284,886, using only preservatives approved for human consumption and pharmaceutical use, including but not limited to potassium sorbate, sorbic acid, benzalkonium chloride, and parabens.

### Example 1: Preparation of a mouthrinse composition containing an antimicrobial agent

A first phase (Phase A) was prepared by combining 120 g of a 1% aqueous solution of oat beta glucan (Ceapro Inc.), 90 g of deionized water, 0.21 g of the sweetener Sunett^{®} (Nutrinova Inc.; chemical name: Acesulfame K), and colouring (blue/green to produce turquoise colour), as required. All of the ingredients of Phase A were mixed until a homogeneous solution was formed. A second phase (Phase B) was prepared by dissolving 0.109 g cetylpyridinium chloride (CPC) USP (Sigma) in 2.0 g warm (45°C) deionized water. Phases A and B were then mixed together to form Phase C. 1.68 g ATMOS 300 (ICI Surfactants), 4.2 g Fresh Mint oil toothpaste flavouring, 2.1 g Spearmint oil, and 0.42 g Cool Artificial Flavouring with Physcool (VMF Mane), were then added, in the order listed to Phase C to form Phase D. An emulsion of Phase D was produced using a homogenizer or highspeed blender for 15-30 secs. The emulsion produced was stable for up to one month. The above formulation was used in a pump spray bottle and applied directly into the mouth.

### Example 2A. Preparation of a toothpaste composition

Oat Beta Glucan Liquid (Ceapro Inc.) was mixed with a commercial toothpaste preparation at a concentration of 10% w/w under emulsifying conditions to produce a toothpaste that provided a clean lubricating feel in the mouth after being used.

### Example 2B. Preparation of a toothpaste composition containing an antibacterial agent

Oat Beta Glucan Liquid (Ceapro Inc.) was mixed with a commercial antibacterial toothpaste preparation at a concentration of 10% w/w under emulsifying conditions to produce a toothpaste that provided a clean lubricating feel in the mouth after being used.

### Example 3. Preparation of a mouthwash or spray containing an avenanthramide-containing extract.

1 g of an oat extract containing 100 pm avenanthramide (Ceapro Inc.) was added to a stirred 10 % (w/w) solution of oat β (1-3) β (1-4) glucan (Ceapro Inc.) producing a clear near-colourless solution.

### Example 4. Preparation of a mouthwash or spray containing a nutraceutical extract

An extract of *Echinacea angustifolia* (1000 mg) in 45% ethanol was added to a stirred solution of oat beta glucan (Ceapro Inc.) to achieve a final concentration of 0.5 % w/w oat beta glucan. The mixture was evaporated under reduced vacuum to remove the alcohol, affording a clear light amber solution.

## Claims

1. A toothpaste for imparting fresh breath to a subject over a prolonged period of time, comprising:
an effective amount of a β (1-3) β (1-4) glucan;
an effective amount of an antibacterial agent;
an effective amount of a flavouring agent;
an effective amount of a surfactant, and
an effective amount of a polishing agent.

2. The toothpaste according to claim 1, wherein the toothpaste comprises:
an effective amount of a β (1-3) β (1-4) glucan;
an effective amount of an antibacterial agent;
an effective amount of a flavouring agent;
an effective amount of a surfactant;
an effective amount of a polishing agent, and
an effective amount of a fluoride salt.

3. The toothpaste according to claim 1, wherein the toothpaste comprises:
an effective amount of a β (1-3) β (1-4) glucan;
an effective amount of an antibacterial agent;
an effective amount of a flavouring agent;
an effective amount of a surfactant;
an effective amount of a sweetener;
an effective amount of a polishing agent, and
an effective amount of a fluoride salt.

4. The toothpaste according to claim 1, 2 or 3, wherein the antibacterial agent is selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.

5. A mouthrinse for imparting fresh breath to a subject over a prolonged period of time, comprising:
an effective amount of a β (1-3) β (1-4) glucan;
an effective amount of an antibacterial agent;
an effective amount of a flavouring agent;
an effective amount of a surfactant, and
an effective amount of a sweetener.

6. The mouthrinse according to composition of claim 5, wherein the antibacterial agent is selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.

7. A tooth-whitening composition comprising:
an effective amount of a β (1-3) β (1-4) glucan, and
an effective amount of a bleaching agent.

8. The tooth-whitening composition according to claim 7, further comprising an effective amount of one, or more than one compound selected from the group consisting of a flavouring agent, an antibacterial agent, a botanical extract, a surfactant, a humectant, a thickener, a fluoride salt, an odour neutralizing agent, an antioxidant, and a polishing agent.

9. The tooth-whitening composition according to claim 7, comprising:
an effective amount of a β (1-3) β (1-4) glucan;
an effective amount of a flavouring agent, and
an effective amount of a bleaching agent.

10. The tooth-whitening composition according to claim 7, comprising:
an effective amount of a β (1-3) β (1-4) glucan;
an effective amount of a flavouring agent;
an effective amount of an antibacterial agent, and
an effective amount of a bleaching agent.

11. A mouthrinse for imparting fresh breath to a subject over a prolonged period of time, comprising:
an effective amount of a β (1-3) β (1-4) glucan, and
an effective amount of an odour neutralizing agent.

12. The mouthrinse according to claim 11, further comprising an effective amount of one, or more than one compound selected from the group consisting of a flavouring agent, an antibacterial agent, a botanical extract, a surfactant, a humectant, a thickener, a fluoride salt, a bleaching agent, an antioxidant, and a polishing agent.

13. The mouthrinse according to claim 11, wherein said odour neutralizing agent is selected from the group consisting of zinc gluconate, zinc citrate, alpha ionone, and a mixture thereof.

14. A mouthrinse for imparting fresh breath to a subject over a prolonged period of time, comprising:
an effective amount of a β (1-3) β (1-4) glucan;
an effective amount of an antibacterial agent selected from the group consisting of triclosan, cetyl pyridinium chloride, sanguinarine, domiphen bromide, a quaternary ammonium salt, a zinc compound, a fluoride, alexidine, octonideine, EDTA, silver nitrate, thymol, methyl salicylate, eucalyptol, menthol, and a mixture thereof.

15. The mouthrinse according to claim 14, further comprising an effective amount of one, or more than one compound selected from the group consisting of a flavouring agent, a polishing agent, a surfactant, a botanical extract, a humectant, a thickener, a fluoride salt, a bleaching agent, a gum base, an antioxidant, and an emulsifier.

16. A method for imparting fresh breath to a subject over a prolonged period of time, comprising applying to the teeth, the oral cavity, or both of a subject, a composition comprising:
an effective amount of a β (1-3) β (1-4) glucan, and
an effective amount of one, or more than one of an antibacterial agent, a botanical extract, and a flavouring agent.

17. The method according to claim 16, wherein the composition further comprises an effective amount of one, or more than one compound selected from the group consisting of a surfactant, a sweetener, a polishing agent, a thickener, a fluoride salt, a bleaching agent, a humectant, an odour neutralizing agent, an antioxidant, and a gum base.

18. A method of whitening the teeth of a subject, comprising applying to the teeth of the subject an oral composition comprising:
an effective amount of a β (1-3) β (1-4) glucan, and
an effective amount of a bleaching agent.

19. The method according to claim 18, wherein the oral composition further comprises an effective amount of one, or more than one compound selected from the group consisting of a flavouring agent, an antibacterial agent, a botanical extract, a surfactant, a humectant, a thickener, a fluoride salt, an antioxidant, and a polishing agent.

## Patentansprüche

1. Zahncreme, um einer Person über einen längeren Zeitraum einen frischen Atem zu verleihen, welche umfasst:
eine wirksame Menge eines β (1-3)- und β (1-4)-Glukans;
eine wirksame Menge eines antibakteriellen Stoffes;
eine wirksame Menge eines Aromastoffes;
eine wirksame Menge eines Tensides; und
eine wirksame Menge eines Poliermittels.

2. Zahncreme nach Anspruch 1, wobei die Zahncreme umfasst,:
eine wirksame Menge eines β (1-3)- und β (1-4)-Glukans;
eine wirksame Menge eines antibakteriellen Stoffes;
eine wirksame Menge eines Aromastoffes;
eine wirksame Menge eines Tensides;
eine wirksame Menge eines Poliermittels; und
eine wirksame Menge eines Fluoridsalzes.

3. Zahncreme nach Anspruch 1, wobei die Zahncreme umfasst:
eine wirksame Menge eines β (1-3)- und β (1-4)-Glukans;
eine wirksame Menge eines antibakteriellen Stoffes;
eine wirksame Menge eines Aromastoffes;
eine wirksame Menge eines Tensides;
eine wirksame Menge eines Süßstoffen;
eine wirksame Menge eines Poliermittels; und
eine wirksame Menge eines Fluoridsalzes.

4. Zahncreme nach Anspruch 1, 2 oder 3, wobei der antibakterielle Stoff ausgewählt wird aus der Gruppe bestehend aus Triclosan, Cetylpyridiniumchlorid, Sanguinarin, Domiphenbromid, einem quarternären Ammoniumsalz, einer Zinkverbindung, einem Fluorid, Alexidin, Octonidein, EDTA, Silbernitrat, Thymol, Methylsalicylat, Eucalyptol, Menthol und einem Gemisch davon.

5. Mundspülung, um einer Person über einen längeren Zeitraum einen frischen Atem zu verleihen, welche umfasst:
eine wirksame Menge eines β (1-3)- und β (1-4)-Glukans;
eine wirksame Menge eines antibakteriellen Stoffes;
eine wirksame Menge eines Aromastoffes;
eine wirksame Menge eines Tensides; und
eine wirksame Menge eines Süßstoffes.

6. Mundspülung gemäß der Zusammensetzung nach Anspruch 5, wobei der antibakterielle Stoff ausgewählt wird aus der Gruppe bestehend aus Triclosan, Cetylpyridiniumchlorid, Sanguinarin, Domiphenbromid, einem quarternären Ammoniumsalz, einer Zinkverbindung, einem Fluorid, Alexidin, Octonidein, EDTA, Silbernitrat, Thymol, Methylsalicylat, Eucalyptol, Menthol und einem Gemisch davon.

7. Zahnaufhellende Zusammensetzung, welche umfasst:
eine wirksame Menge eines β (1-3)- und β (1-4)-Glukans;
eine wirksame Menge eines Bleichmittels.

8. Zahnaufhellende Zusammensetzung nach Anspruch 7, die ferner eine wirksame Menge einer oder mehrerer Verbindungen umfasst, ausgewählt aus der Gruppe bestehend aus einem Aromastoff, einem antibakteriellen Stoff, einem Pflanzenextrakt, einen Tensid, einem Feuchthaltemittel, einem Verdickungsmittel, einem Fluoridsalz, einem Mittel zur Geruchsneutralisierung, einen Antioxidationsmittel und einem Poliermittel.

9. Zahnaufhellende Zusammensetzung nach Anspruch 7, welche umfasst:
eine wirksame Menge eines β (1-3)- und β (1-4)-Glukans;
eine wirksame Menge eines Aromastoffes; und
eine wirksame Menge eines Bleichmitlels.

10. Zahnaufhellende Zusammensetzung nach Anspruch 7, welche umfasst:
eine wirksame Menge eines β (1-3)- und β (1-4)-Glukans;
eine wirksame Menge eines Aromastoffes;
eine wirksame Menge eines antibakteriellen Stoffes; und
eine wirksame Menge eines Bleichmittels.

11. Mundspülung, um einer Person über einen längeren Zeitraum einen frischen Atem zu verleihen, welche umfasst:
eine wirksame Menge eines β (1-3)- und β (1-4)-Glukans;
eine wirksame Menge eines Mittels zur Geruchsneutralisierung.

12. Mundspülung nach Anspruch 11, die ferner eine wirksame Menge einer oder mehrerer Verbindungen umfasst, ausgewählt aus der Gruppe bestehend aus einem Aromastoff, einem antibakteriellen Stoff, einem Pflanzenextrakt, einem Tensid, einem Feuchthaltemittel, einem Verdickungsmittel, einem Fluoridsalz, einem Bleichmittel, einem Antioxidationsmittel und einem Poliermittel.

13. undspülung nach Anspruch 11, wobei das Mittel zur Geruchsneutralisierung ausgewählt wird aus der Gruppe bestehend aus Zinkglukonat, Zinkcitrat, Alphaionon und einem Gemisch davon.

14. Mundspülung, um einer Person über einen längeren Zeitraum einen frischen Atem zu verleihen, welche umfasst:
eine wirksame Menge eines β (1-3)- und β (1-4)-Glukans;
eine wirksame Menge eines antibakteriellen Stoffes, ausgewählt aus der Gruppe bestehend aus Triclosan, Cetylpyridiniumchlorid, Sanguinarin, Domiphenbromid, einem quarternären Ammoniumsalz, einer Zinkverbindung, einem Fluorid, Alexidin, Octonidein, EDTA, Silbernitrat, Thymol, Methylsalicylat, Eucalyptol, Menthol und einem Gemisch davon.

15. Mundspülung nach Anspruch 14, die ferner eine wirksame Menge einer oder mehrerer Verbindungen umfasst, ausgewählt aus der Gruppe bestehend aus einem Aromastoff, einem Poliermittel, einem Tensid, einem Pflanzenextrakt, einem Feuchthaltemittel, einem Verdickungsmittel, einem Fluoridsalz, einem Bleichmittel, einer Kaugummibase, einem Antioxidationsmittel und einem Emulgiermittel.

16. Verfahren, um einer Person über einen längeren Zeitraum einen frischen Atem zu verleihen, welches das Anwenden einer Zusammensetzung auf Zähne, Mundhöhle oder beides einer Person umfasst, wobei die Zusammensetzung umfasst:
eine wirksame Menge eines β (1-3)- und β (1-4)-Glukans; und
eine wirksame Menge mindestens eines antibakteriellen Stoffes, eines Pflanzenextraktes oder eines Aromastoffes.

17. Verfahren nach Anspruch 16, wobei die Zusammensetzung ferner eine wirksame Menge einer oder mehrerer Verbindungen umfasst, ausgewählt aus der Gruppe bestehend aus einem Tensid, einem Süßstoff, einem Poliermittel, einem Verdickungsmittel, einem Fluoridsalz, einem Bleichmittel, einem Feuchthaltemittel, einem Mittel zur Geruchsneutralisierung und einer Kaugummibase.

18. Verfahren zum Aufhellen der Zähne einer Person, welches das Auftragen einer oralen Zusammensetzung auf die Zähne der Person umfasst, welche umfasst:
eine wirksame Menge eines (1-3)- und (1-4)-Glukans;
eine wirksame Menge eines Bleichmittels.

19. Verfahren nach Anspruch 18, wobei die orale Zusammensetzung ferner eine wirksame Menge einer oder mehrerer Verbindungen umfasst, ausgewählt aus der Gruppe bestehend aus einem Aromastoff, einem antibakteriellen Stoff, einem Pflanzenextrakt, einem Tensid, einem Feuchthaltemittel, einem Verdickungsmittel, einem Fluoridsalz, einem Antioxidationsmittel und einem Poliermittel.

## Revendications

1. Pâte dentifrice pour conférer une haleine fraîche à un sujet pendant une période de temps prolongée, comprenant :
une quantité efficace d'un β (1-3) β (1-4) glucane ;
une quantité efficace d'un agent antibactérien ;
une quantité efficace d'un agent aromatisant ;
une quantité efficace d'un agent tensio-actif ; et
une quantité efficace d'un agent de polissage.

2. Pâte dentifrice selon la revendication 1, dans lequel le dentifrice comprend :
une quantité efficace d'un β (1-3) β (1-4) glucane ;
une quantité efficace d'un agent antibactérien ;
une quantité efficace d'un agent aromatisant ;
une quantité efficace d'un agent tensioactif ;
une quantité efficace d'un agent de polissage ; et
une quantité efficace d'un sel fluorure.

3. Pâte dentifrice selon la revendication 1, dans lequel le dentifrice comprend :
une quantité efficace d'un β (1-3) β (1-4) glucane ;
une quantité efficace d'un agent antibactérien ;
une quantité efficace d'un agent aromatisant ;
une quantité efficace d'un agent tension-actif
une quantité efficace d'un agent édulcorant ;
une quantité efficace d'un agent de polissage ; et
une quantité efficace d'un sel fluorure.

4. Pâte dentifrice selon l'une des revendications 1, 2 ou 3, dans lequel l'agent antibactérien est choisi dans le groupe consistant en le triclosan, le chlorure de cétyl pyridinium, la sanguinarine, le bromure de domiphène, un sel d'ammonium quaternaire, un composé du zinc, un fluorure, l'alexidine, l'octonidéine, l'EDTA, le nitrate d'argent, le thymol, le salicylate de méthyle, l'eucalyptol, le menthol et un mélange de ceux-ci.

5. Bain de bouche pour conférer une haleine fraîche à un sujet pendant une période de temps prolongée, comprenant :
une quantité efficace d'un β (1-3) β (1-4) glucane ;
une quantité efficace d'un agent antibactérien ;
une quantité efficace d'un agent aromatisant ;
une quantité efficace d'un agent tensio-actif ; et
une quantité efficace d'un agent édulcorant.

6. Bain de bouche selon la composition de la revendication 5, dans lequel l'agent antibactérien est choisi dans le groupe consistant en le triclosan, le chlorure de cétyl pyridinium, la sanguinarine, le bromure de domiphène, un sel d'ammonium quaternaire, un composé du zinc, un fluorure, l'alexidine, l'octonidéine, l'EDTA, le nitrate d'argent, le thymol, le salicylate de méthyle, l'eucalyptol, le menthol et un mélange de ceux-ci.

7. Composition de blanchiment des dents comprenant :
une quantité efficace d'un β (1-4) glucane ; et
une quantité efficace d'un agent de blanchiment.

8. Composition de blanchiment des dents selon la revendication 7, comprenant en outre une quantité efficace d'un ou de plus d'un composé choisi dans le groupe consistant en un agent aromatisant, un agent antibactérien, un extrait botanique, un agent tensio-actif, un humectant, un épaississant, un sel fluorure, un agent neutralisant les odeurs, un anti-oxydant et un agent de polissage.

9. Composition de blanchiment des dents selon la revendication 7, comparenant:
une quantité efficace d'un β (1-3) β (1-4) glucane ;
une quantité efficace d'un agent aromatisant ; et
une quantité efficace d'un agent de blanchiment.

10. Composition de blanchiment des dents selon la revendication 7, comprenant :
une quantité efficace d'un β (1-3) β (1-4) glucane ;
une quantité efficace d'un agent aromatisant ;
une quantité efficace d'un agent antibactérien ; et
une quantité efficace d'un agent de blanchiment.

11. Bain de bouche pour conférer une haleine fraîche à un sujet pendant une période de temps prolongée, comprenant :
une quantité efficace d'un β (1-3) β (1-4) glucane ; et
une quantité efficace d'un agent neutralisant les odeurs.

12. Bain de bouche selon la revendication 11, comprenant en outre une quantité efficace d'un ou plus d'un composé choisi dans le groupe consistant en un agent aromatisant, un agent antibactérien, un extrait botanique, un agent tensio-actif, un humectant, un épaississant, un sel fluorure, un agent de blanchiment, un anti-oxydant et un agent de polissage.

13. Bain de bouche selon la revendication 11, dans lequel ledit agent neutralisant les odeurs est choisi dans le groupe consistant en le gluconate de zinc, le citrate de zinc, l'alpha-ionone et un mélange de ceux-ci.

14. Bain de bouche pour conférer une haleine fraîche à un sujet pendant une période de temps prolongée, comprenant :
une quantité efficace d'un β (1-4) glucane ;
une quantité efficace d'un agent antibactérien choisi dans le groupe consistant en le triclosan, le chlorure de cétyl pyridinium, la sanguinarine, le bromure de domiphène, un sel d'ammonium quaternaire, un composé du zinc, un fluorure, l'alexidine, l'octonidéine, l'EDTA, le nitrate d'argent, le thymol, le salicylate de méthyle, l'eucalyptol, le menthol et un mélange de ceux-ci.

15. Bain de bouche selon la revendication 14, comprenant en outre une quantité efficace d'un ou plus d'un composé choisi dans le groupe consistant en un agent aromatisant, un agent de polissage, un agent tensio-actif, un extrait botanique, un humectant, un épaississant, un sel fluorure, un agent de blanchiment, une base de gomme, un anti-oxydant et un émulsifiant.

16. Procédé pour conférer une haleine fraîche à un sujet pendant une période de temps prolongée, comprenant l'application aux dents, à la cavité buccale, ou aux deux, d'un sujet, d'une composition comprenant :
une quantité efficace d'un β (1-3) β (1-4) glucane ; et
une quantité efficace d'un ou de plus d'un parmi un agent antibactérien, un extrait botanique et un agent aromatisant.

17. Procédé selon la revendication 16, dans lequel la composition comprend en outre une quantité efficace d'un ou de plus d'un composé choisi dans le groupe consistant en un agent tensio-actif, un agent édulcorant, un agent de polissage, un épaississant, un sel fluorure, un agent de blanchiment, un humectant, un agent neutralisant les odeurs, un anti-oxydant et une base de gomme.

18. Procédé de blanchiment des dents d'un sujet, comprenant l'application aux dents du sujet d'une composition orale comprenant :
une quantité efficace d'un β (1-3) β (1-4) glucane ; et
une quantité efficace d'un agent de blanchiment.

19. Procédé selon la revendication 18, dans lequel la composition orale comprend en outre une quantité efficace d'un ou de plus d'un composé choisi dans le groupe consistant en un agent aromatisant, un agent antibactérien, un extrait botanique, un agent tensio-actif, un humectant, un épaississant, un sel fluorure, un anti-oxydant et un agent de polissage.
